# EUROPEAN PATENT APPLICATION

(11) **EP 4 480 392 A1**
(43) Date of publication of application: **25.12.2024**
(21) Application number: 23180255.4
(22) Date of filing: 20.06.2023
(51) Int. Cl.: A61B 5/00

(54) **TISSUE IMAGING**

(71) Applicant: Koninklijke Philips N.V., 5656 AG Eindhoven (NL)
(72) Inventor: PERRONE, Antonio Luigi, Eindhoven (NL); JOHNSON, Mark Thomas, Eindhoven (NL); GERHARDT, Lutz Christian, Eindhoven (NL)
(74) Representative: Philips Intellectual Property & Standards

(57) **Abstract**

Proposed concepts aim to provide schemes, solutions, concepts, designs, methods, and systems pertaining to the imaging of tissue of a subject. Specifically, polarized light is emitted toward tissue of the subject, and the light backscattered and/or transmitted by the tissue is detected through a filter different polarized to the emitted light. The detected light is used to generate an image of the tissue of the subject. Put simply, better depth penetration of the tissue may be realised by use of polarized light. Further, the use of polarized light to image the tissue provides an integrated view of the structural properties of the tissue. This may be particularly beneficial, for example, for breast and/or nipple tissue imaging in which calcifications can form at depths difficult to image by typical optical methods.

## Description

### FIELD OF THE INVENTION

The present invention relates to the field of imaging tissue of a subject using light.

### BACKGROUND OF THE INVENTION

In recent years, breast and nipple anatomy has become of growing interest due to its increasing clinical relevance. Indeed, this anatomy is related to a number of small and large pathologies, mostly for the female population. Such pathologies range from blocked ducts in the nipple to tumorous calcifications in the breast tissue. Presently, diagnostic techniques can access the internal breast through nipple ducts and surgeons can offer nipple-sparing mastectomy. However, there is variation in the number of ducts present in the breast and their spatial location is challenging to visualize. The best-known visualization techniques relate to the use of ex-vivo tissues typically obtained via mastectomies.

Put another way, visualizing the totality of ducts is highly relevant for diagnosis of various pathologies, but proves challenging. In particular, there are serious deficiencies in in-vivo visualization techniques. Often, the development and change of characteristics of nipple and breast tissue is important for accurate and timely diagnosis of pathologies. However, deep tissue imaging of the breast performed by radiographic techniques, or (more recently) by ultrasound. Thus, it is difficult to realize the benefits associated with periodic and detailed imaging of the breasts for the early warning of suspected tumors.

Therefore, there is a need for a simple and cost-effective technique for observation and imaging. In particular, it would be particularly beneficial if such a technique could be integrated into the current medical protocols for self-observation and reporting, especially at home.

### SUMMARY OF THE INVENTION

The invention is defined by the claims.

According to examples in accordance with an aspect of the invention, there is provided a system for imaging tissue of a subject, comprising:
a light source configured to emit monochromatic light toward the tissue of the subject;
a first polarization filter configured to polarize light emitted by the light source, and to transmit polarized light toward the tissue of the subject;
a second polarization filter configured to filter light backscattered and/or transmitted by the tissue, and to transmit the filtered light, wherein the filtered light is differently polarized compared to the emitted light;
a light sensor configured to detect the filtered light;
a controller configured to control the light source to emit first light having a first wavelength, and control the light sensor to detect first filtered light corresponding to the first light; and
a processor configured to process the first filtered light to generate an image of the tissue of the subject.

By using polarized light to image the tissue of the subject enables a deeper imaging of the tissue and the polarized light can penetrate deeper into the tissue of the subject. Accordingly, the invention provides a relatively simple and affordable (due to the lack of custom, complicated and/or expensive parts) way to image tissue of the subject. Indeed, the system may be integrated into a personal care device to facilitate fast and simple imaging, thereby improving the potential frequency of imaging sessions and accessibility to perform such imaging sessions away from medical supervision.

In other words, polarization filters are used firstly to simply polarize emitted light, and then polarize the light (after being reflected/backscattered and/or transmitted by the tissue) differently. This enables deep (sub-surface) imaging of the tissue that can be used to roughly identify structures in the tissue. Subsequent analysis, parameter alteration and repeated measurements may be acquired to overcome potential spatial resolution problems.

Indeed, typically complex and costly optics are required to perform optical imaging of (bulk/below surface) imaging of the subject. Such complicated systems may be used to obtain images of high spatial resolution and quality, but require precise aligning, expensive optics and do not penetrate as deeply into the tissue. The proposed invention overcomes these issues with the usual optical systems, and post-processing and changes (informed by feedback) used to improve image resolution and quality.

Furthermore, different types of tissues will interact with polarized light different. That is, soft tissue may change the polarization characteristics of polarized light differently to tumors or calcifications within the tissue. Therefore, by detecting backscattered and/or reflected light through a polarization filter (with different polarization characteristics than the polarization filter used to polarize the emitted/produced light), the resultant interaction of polarization by the tissue can be appreciated, and used to identify structures in the tissue.

In some embodiments, the controller may be further configured to control the light source to emit second light having a second wavelength different from the first wavelength, and control the light sensor to detect second filtered light corresponding to the second light. In this case, the processor is further configured to process the first filtered light and the second filtered light to generate the image of the tissue of the subject.

One means by which spatial resolution and quality of the image of the tissue of the subject can be improved is by acquiring two images by lights of different frequencies. This is known as spatial polarization difference imaging (SPDI), and essentially uses two different frequencies to illuminate the biological tissue, acquiring polarization images from both frequencies. A final image may have an improved signal to noise ratio.

Specifically, the controller may be further configured to determine the second wavelength different from the first wavelength based on a predetermined sequence of wavelengths.

Each wavelength (and their relative differences) of the selected light emitted/used to illuminate the tissue will impact the resultant image quality. Therefore, a sequence of wavelengths may be followed to determine the best combination of wavelengths. As a result, an image with an improved signal to noise ratio may be obtained.

More particularly, the processor may be configured to process the first filtered light to generate a first intermediate image of the tissue of the subject, process the second filtered light to generate a second intermediate image of the tissue of the subject, and generate the image of the tissue of the subject based on a difference between the first intermediate image and the second intermediate image.

In some embodiments, the processor is further configured to process the first filtered light to generate Stokes vectors representing the polarization of the first filtered light, and analyse the Stokes vectors using Mueller matrix decomposition to determine a characteristic value describing a property of the tissue of the subject.

Mueller matrix decomposition provides an effective tool for simply analysing a representation of the polarization of the detected light - thus providing information as to the tissue properties of the tissue that the light was backscattered from and/or transmitted by. Such information may be used to determine parameters for future imaging scans, or may be useful for identification of certain structures and/or pathologies.

Moreover, the first polarization filter may be adapted to be operable in a plurality of first polarization modes, wherein a polarization characteristic of the transmitted polarized light is different in each first polarization mode. Similarly, the second polarization filter may be adapted to be operable in a plurality of second polarization modes, wherein a polarization characteristic of the filtered light is different in each second polarization mode.

As the polarization characteristics of both of the polarization filters may be changed, an appropriate polarization for the particular tissue being imaged may be implemented. Moreover, as the mode of operation of the filters may be changed, multiple images of the same tissue with different polarizations may be obtained, and an overall horizontal resolution of the image of the tissue improved.

In some embodiments, the light source may comprise a light emitter configured to emit light in the infrared, near-infrared, and/or visible light spectrums, and a variable light frequency filter configured to receive the emitted light, and output monochromatic light toward the tissue of the subject. In this case, the controller may be configured to control the variable light frequency filter to output monochromatic light having the first wavelength as the first light.

A light source suitable for implementing this embodiment of the invention may be realised by relatively simple and inexpensive components (light emitter, such as an LED, and a light frequency filter). This also enables the selection of different wavelengths of light with one light source, facilitating SPDI and improving resultant image quality.

Furthermore, the processor in some embodiments may be further configured to process the first filtered light to determine an object detection value indicative of the presence of an object within the tissue of the subject.

This enables the automatic detection of objects without the need for operation by a skilled individual, thus improving the usability of the system (especially for home-use). Indeed, once detected, the system may automatically focus on areas where an object is detected, ensuring adequate imaging is performed for that area.

More particularly, the control unit may be configured to, responsive to the object detection value determined by the processor meeting a predetermined requirement: control the light source to emit a plurality of lights, each having a different wavelength, and control the light sensor to detect a plurality of filtered lights corresponding to the plurality of lights with different wavelengths. In this case, the processor may be further configured to process the plurality of filtered lights to generate a detailed image of the tissue of the subject, the detailed image having a greater spatial resolution than the image of the tissue of the subject.

An area of interest may benefit from an improved imaging by acquiring a plurality of images with different light wavelengths. As above, this improves the spatial resolution of the image so an improved understanding on a (potential) object (i.e. a tumor or calcification) in the tissue may be realised.

Alternatively, or in addition, in some embodiments the control unit may be configured to, responsive to the object detection value determined by the processor meeting a predetermined requirement: control the light source to emit a plurality of lights, each having a different polarization characteristic, and control the light sensor to detect a plurality of filtered lights corresponding to the plurality of lights with different polarization characteristics. In this case, the processor may be further configured to process the plurality of filtered lights to generate a detailed image of the tissue of the subject, the detailed image having a greater spatial resolution than the image of the tissue of the subject.

Indeed, similarly to the above, once detected it may be beneficial to improve a horizontal resolution of the image associated with the detected object. Once again, this improves an effectiveness of the system and a quality of resultant imaging of the tissue.

In some embodiments, the system may further comprise an analysis unit configured to identify features present in the image of the tissue of the subject, and map the identified features to a model representing an anatomy of the subject including the tissue to generate a subject-specific feature map.

Not only is the detection/imaging the tissue important, but an analysis of the tissue over time may be key. In many cases, changes over time are more important for effective diagnosis and/or detection of pathologies. Thus, by automatically recording and mapping features to a model, an analysis over time can be provided by the invention.

In some cases, the analysis unit may be further configured to compare the subject-specific feature map to a previous subject-specific feature map representing a mapping of previously identified features of the tissue of the subject; and identify a change in features of the subject based on a result of the comparison.

In a preferred embodiment, the tissue may be breast tissue of the subject, and the system may be mounted on a breast pump device.

Breast tissue of the subject is at risk of many different pathologies which may exist deep within the breast tissue. For example, nipple ducts may extend several millimetres beneath the surface and may be subject to blockages. Therefore, the present system may be particularly beneficial for imaging breast tissue due to the system's ability to image deep below the tissues surface.

Moreover, integration of the system into a breast pump may enable a simple and effective means for consistent and at-home imaging of breast tissue of the subject, so as to identify objects and potential pathologies as early as possible (whilst avoiding the need for lengthy, invasive, pre-emptive testing/screening). Indeed, the proposed system is particularly suitable for mounting/integration with a breast pump due to the small and simple elements required to realise.

According to examples in accordance with a further aspect of the invention there is provided a method for imaging tissue of a subject, comprising:
emitting a first light having a first wavelength toward the tissue of the subject, wherein the first light is monochromatic;
filtering the first light such that the first light is polarized;
filtering light that is backscattered and/or transmitted by the tissue, such that the backscattered and/or transmitted light is polarized, wherein the backscattered and/or transmitted light is differently polarized compared to the first light;
detecting the polarized backscattered and/or transmitted light; and
processing the detected light to generate an image of the tissue of the subject.

According to additional examples in accordance with another aspect of the invention there is provided a computer program product comprising computer program code means which, when executed on a computing device having a processing system, cause the processing system to perform all of the steps of the method for imaging tissue of a subject according to an embodiment of the invention.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents a block diagram of a system for imaging tissue of a subject according to an embodiment of the invention;
Fig. 2 presents a flow diagram of a method for imaging tissue of a subject according to another embodiment of the invention;
Fig. 3 presents a flow diagram of a method for controlling the light source and light sensor according to another aspect of the invention; and
Fig. 4 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Proposed concepts aim to provide schemes, solutions, concepts, designs, methods, and systems pertaining to the imaging of tissue of a subject. Specifically, polarized light is emitted toward tissue of the subject, and the light backscattered and/or transmitted by the tissue is detected through a filter different polarized to the emitted light. The detected light is used to generate an image of the tissue of the subject. Put simply, better depth penetration of the tissue may be realised by use of polarized light. Further, the use of polarized light to image the tissue provides an integrated view of the structural properties of the tissue. This may be particularly beneficial, for example, for breast and/or nipple tissue imaging in which calcifications can form at depths difficult to image by typical optical methods.

That is, by using polarized light as opposed to non-polarized light, an increase in optical depth penetration may be achieved. For example, depth penetration may increase from 1-2 mm as in typical optical methods, to 6-8mm when using polarized light. Indeed, depths of 10-15mm polarimetric may be achieved with higher FWHM. This is particularly beneficial for breast tissue, in which structures may develop deep within the tissue, and nipple ducts often being up to 5mm deep.

The invention proposes a tissue imaging method that may be capable of monitoring the variations of relevant anatomical structures. More specifically, the invention may be particularly suitable for imaging of the breast, including nipple ducts and calcifications, in a range of dimensions. Readily available components may be utilized to realize the invention, thereby enabling low-cost implementation of the invention.

In some cases, the invention proposes the use of polarimetric imaging by spectral polarization difference imaging (SPDI), and Mueller matrix imaging, which can be used to characterize bulk tissues. By combination of SPDI with Mueller matrix imaging the signal information content is increase, even with an economic optical setup.

Furthermore, known problems related to polarimetric imaging (e.g., the delivery of the polarized light to the tissue, and the lower image quality) may be overcome simply by the longitudinal use (i.e. many images acquired over time) of the device that allows an increasing quality of the imaging in time. That is, a better S/N ratio may simply be obtained by repeated measurements.

Presently, it is problematic to visualize in-vivo the totality of the ducts in breast and/or nipple tissue. Indeed, such visualization could prove very beneficial, because such tissues play a very important role in the characterization of different conditions (e.g., ductal carcinoma in situ (DCIS) and blocked ducts induced mastitis).

Specifically, for DCIS it is imperative to find imaging technologies in support of the standard mammography techniques of diagnosis and characterisation (especially given the stealthy nature of such a tumor). Indeed, detection of the tumor proves difficult in its early stages, even in mammograms. In fact, DCIS does not have specific symptoms such a lump or breast pain. Most cases are diagnosed in a mammogram before causing any symptoms. DCIS most commonly shows up on a mammogram as a new calcium deposit, but not always. Sometimes, a distortion of the breast tissue on the scan can be a sign of DCIS. DCIS accounts for about 20% of all breast tumors. Thus, an imaging method to aid in the early detection of DCIS may prove invaluable.

Moreover, milk duct blockage can happen both for women in reproductive age and in perimenopause or after menopause. In the case of perimenopause, mammary duct ectasia may occur when one or more milk ducts beneath the nipple widens. The duct walls may thicken, and the duct may fill with fluid. The milk duct may become blocked or clogged with a thick, sticky substance. The condition often causes no symptoms, but some women may have nipple discharge, breast tenderness or inflammation of the clogged duct (periductal mastitis). In some cases, surgical intervention is needed to remove the affected milk duct. It is important to notice the widening effect of this condition on the ducts affected in order to prevent the subsequent adverse impacts.

Of course, clogged milk ducts can develop also during breastfeeding. Milk ducts are tiny tubes that carry milk from glands (lobules) inside the breast out to the nipple. Each nipple has approximatively 15 to 25 tiny openings called pores, but only about 6 or 7 are open to release milk during nursing. Sometimes a duct can get blocked with milk, which may result in an area of the breast becoming red and tender. Some signs of a plugged milk duct can be a lump on the breast, a red and irritated lump, a soft or tender lump, or a feeling that the breasts are full even after breastfeeding. One method to solve the issue with the clogged duct is trying to use a breast pump. However, if the blockage is not resolved, it could degenerate into puerperal mastitis (i.e., a type of inflammation).

Thus, some embodiments of the present invention offer an economical, effective, and safe imaging technique for tissue (for example, breast tissue) able to detect changes at adequate depths. For example, imaging at depths greater than a few millimetres is beneficial for the diagnosis for the above outlined pathologies/conditions. That is, the invention aims to provide a means to facilitate relating physical changes in the structure of the biological tissue in imaged tissue to the above-mentioned pathological conditions. In some cases, the invention may be implemented/included in personal health devices to enable simple (even at-home) detection various conditions.

Focussing on the specific example of imaging of breast/nipple tissue, the position of blockages is often some distance behind the nipple, whereby the swelling may only be visible when imaging some distance behind the nipple. For this reason, a polarized imaging system may be beneficial. The use of polarization increases the effective depth of imaging by at least a factor of 2.

The problem may thus be stated as how to image:
(i) Local variations of the breasts' whole nipples structures (lactating ducts) in an efficient, simple and affordable way. To achieve this, there requires the detection of blockages of the breasts' ducts during the normal operation of a personal health device (mostly, but not exclusively, breast pumps). monitoring and detection of signs of DCIS through monitoring in time of the nipples' ducts environment, and monitoring and detection of signs of Paget disease of the breast, accounting for about 4% of total breast cancer cases and including both women and men populations.
(ii) Global variations of the breasts' inner tissue clusters so to monitor in time their possible enlargement as a precursor signal of tumor like developments.

For the local variations, to enable this type of imaging, the specifics of the involved anatomy should be considered. For example, it is known that nipple ducts have a diameter that changes with the depth of the breast tissue. Indeed, to detect single ducts, the system must have the ability to resolve structures from 0.2mm to 0.8mm of diameter at depths from 2 to 5 mm into the nipple tissue. This range is possible by polarimetric imaging.

Thus, with such imaging capability it is possible to obtain complete 3D imaging of the whole nipple.

For the global variations, it would be beneficial to visualise variations in the breast structure to identify any internal anatomical change in the range of interest. That is, it would be useful to monitor time variations useful to detect possible abnormal physiological development that can be precursors of calcifications or lumps etc. possibly related to breast cancer developments.

In more detail, monitoring the time evolution of dense structures inside the breast tissue that are, roughly, from 4 to 20mm of diameter (corresponding approximately to the T1 stage of breast tumor classification) is achievable by this invention. This detection is within the capability of certain embodiments of the invention up to a few cm, measured perpendicularly from the corresponding skin surface, inside the breast tissue. Similarly, embodiments of the invention may be able to find traces of structures at the same, or greater (around 3cm) depth, for tumors having a size in the range of the T2 (2-5cm) and T3 (>5 cm) stages.

More generally, the proposed invention utilises polarization, due to associated advantages in implementation, cost and depth penetration in biological tissues. An electromagnetic wave (e.g., light) consists of a coupled oscillating electric field and magnetic field that are always perpendicular to each other. The polarization of light refers to the direction of the electric field. In linear polarization, the fields oscillate in a single direction. In circular or elliptical polarization, the fields rotate at a constant rate in a plane as the wave travels.

Imaging with polarized light is particularly suited to the characterization/imaging of tissue as needed for the diagnosis of various pathologies as outlined above, since it allows:
(i) a better depth penetration in the biological tissue;
(ii) an integrated view of the structural properties; and
(iii) a substantially wider acquisition angle compared to optical methods.

However, polarization-based imaging usually has limited resolution in comparison to optical, depth defined, methods. Although, it should be noted that the gap in resolution with optical depth defined methods may be resolved by focusing on limited volumes of tissue to be imaged and/or using advanced computational methods to enhance the sensibility of the polarimetric method. Indeed, this may be particularly of interest in the application of the invention to imaging of the lactating ducts of a nipple (which may be relatively deep below the tissue surface but have a small size).

Most bulk tissue polarimetric methods may yield well-resolved lateral images but offer cumulative polarization properties through the interrogated depth direction. Further, bulk tissue polarimetric methods have the advantage of being less complex, less expensive, sensitive to larger sampling depths, and easier and faster to implement for a large lateral field of view than the depth-resolved imaging modalities. All these properties match perfectly with the cost and realization simplicity requirements of any personal healthcare device.

The cumulative integration of a polarization imaging method allows for deeper analysis into the tissue, which is of no impediment in a personal healthcare context since the aim is to monitor and indicate suspected abnormal internal structures through comparison of similar images at different times, and not define exactly the geometrical properties (size, volume, depth etc.) of identified changes in the tissue.

Moreover, the periodical use (i.e., use over time) of a device for polarization imaging allows for iterative reduction of the S/N ratios in the imaging measurements due to repetition statistics.

Of course, various techniques may be used to improve tissue imaging based on polarized light. Specifically, Mueller matrix decomposition may be used, which is a matrix method for manipulating Stokes vectors (that represent the polarization of light). In summary, the interaction of the polarized light with the tissue by collecting the backscattered light and/or transmitted light can be analysed to deduce the components and consequently the associated tissue properties.

Furthermore, spectral polarization difference imaging (SPDI) may be performed, which uses two different frequencies of light to illuminate the biological tissue, acquiring polarization images from both of them. The final image derives from the subtraction of the two images acquired at different frequencies so to improve the signal to noise of the resulting image.

Accordingly, a specific realisation of the invention may be embodied in a visual, near infrared and/or infrared (VISNIR) illumination and imaging device mounted on a personal healthcare device (e.g., a breast pump, massage device), the illumination and imaging device comprising:
An illumination part (i.e., a light source) including:
   (i) A broadband VISNIR light source/s (e.g., LED);
   (ii) A programmable VISNIR light frequency filter (monochromator i.e., Fabry-Perot interferometer etc.);
   (iii) A programmable polarization filter (i.e., variable polarizer)
An imaging component (i.e., light sensor) including:
   (i) A CMOS sensor without an IR-NIR filter applied;
   (ii) Lenses for image focusing (i.e., variable focus lenses etc.);
   (iii) A programmable polarization filter (i.e., variable polarizer)
A processor including:
   (i) A component for estimating/determining the presence of tissue (breast/s, nipple) in the field of view of the CMOS camera;
   (ii) A component for implementing SPDI;
   (iii) A component for implementing Mueller matrix decomposition imaging.

A control module for implementing the progressive multi-polarization and multi-frequency imaging of the imaged tissue, and of the eventually evidenced solid objects/calcifications.

In some cases, the control module may first implement a first (rough) estimation of the VISNIR reflectance (using results of the SPDI and Mueller matrix decomposition) to evaluate presence of objects inside the tissue (e.g., calcifications inside the breast), of principal axis greater or equal to 2-4mm.

Then, the control module may gather results of SPDI and Mueller matrix decomposition at multiple frequencies and polarizations. This may ensure for details smaller than 2mm and/or better depth resolution of the identified objects is obtained.

The control module may also sample more images in areas of high likelihood of objects. For example, sample of images of the breast may be increased in areas of higher tumoral prevalence.

Subsequently, a 3D volume reconstruction from 2D images acquired during the presence estimation process may be generated, and a 3D mapping of the imaged structures to the tissue performed. This may allow longitudinal integration and analysis of the tissue.

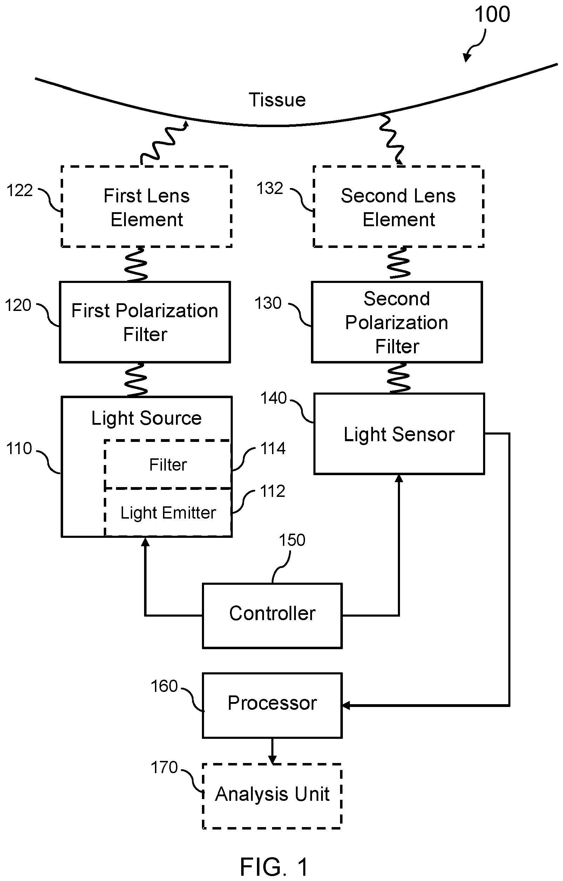

Moving onto Fig. 1, there is depicted a simplified block diagram of a system for imaging tissue of a subject according to an embodiment of the invention. The system comprises a light source 110, a first polarization filter 120, a second polarization filter 130, a light sensor 140, a controller 150, and a processor 160. Optionally, the system further comprises a first and second lens element 122, 132, and analysis unit 170.

The system is configured so as to be able to produce an image of the (bulk) tissue of the subject. That is, the system may be used to produce an image of a volume beneath the surface of skin of the subject, such as breast tissue, nipple tissue or muscle tissue.

By way of specific example, the tissue may be breast and/or nipple tissue of the subject. Furthermore, in this case, the system may be mounted on a breast pump device. This means that imaging may be performed at regular intervals without necessity for separate equipment and time outside of a breast pumping scenario.

The light source 110 is configured to emit monochromatic light toward the tissue of the subject. That is, the light source 110 is configured to irradiate tissue of the subject with monochromatic light. Monochromatic light in this context means light of a single wavelength, or a narrow band of wavelengths.

For example, the light source 110 may comprise a broadband light emitter (e.g. an LED) with a light filter allowing only monochromatic light from the broadband light emitter. The light filter, in some cases, be operable in multiple modes to let monochromatic light of different wavelengths through. Alternatively, the light source 110 may simply comprise a light emitter configured to emit monochromatic light.

More particularly, wherein the light source 110 may (optionally) comprise a light emitter 112 configured to emit light in the infrared, near-infrared, and/or visible light spectrums, and a variable light frequency filter 114 configured to receive the emitted light and output monochromatic light toward the tissue of the subject.

In any case, the light source 110 may emit light in the visible, infrared, and/or near infrared spectrums. The emission of light toward tissue of the subject may simply mean that the light source 110 provides light to suitable optics for directing light from the light source 110 toward a surface of the subject. Alternatively, the light source 110 may be configured such that the light is directly emitted toward the tissue (via the first polarization filter 120).

To reiterate, it may be the case that the light source 110 can (selectively) emit different monochromatic light. That is, light of different wavelengths may be emitted by the light source 110 selectively. This enables multi-wavelength imaging for selective depths and enables SPDI.

The light sensor 140 is configured to detect light (corresponding to the emitted light) backscattered (i.e., reflected) and/or transmitted by the tissue of the subject. The light sensor 140, for example, may be a CMOS sensor or other camera system that can detect light (in at least one of the visible, infrared, and near infrared spectrums). Essentially, the light sensor 140 may be any component that can convert incident light into a signal describing the incident light for further analysis.

The light sensor 140 may be appropriately positioned to detect the backscattered and/or transmitted light. In some cases, it may be preferable that the light sensor 140 is positioned to only detect backscattered light, which lends itself to a more compact system (i.e., does not require the sensor and source to be positioned opposite sides of the tissue). The light sensor 140 may comprise suitable optics for gathering/guiding light toward a component for sensing the light.

Further to the light source 110 and light sensor 140, the system further comprises a first polarization filter 120 and second polarization filter 130 for implementing polarization-based imaging.

Specifically, the first polarization filter 120 is configured to polarize/filter light emitted by the light source 110, and to transmit polarized light toward the tissue of the subject. That is, the light from the light source 110 passes through the first polarization filter 120 before illuminating the tissue. The first polarization filter 120 polarizes light in a first mode (e.g., horizontally, vertically, right circular or left circular).

The second polarization filter is configured to polarize light after backscattering and/or transmission by the tissue of the subject, and to transmit the resultant polarized light to the light sensor 140. In other words, the second polarization filter 130 is configured to filter/polarize light backscattered and/or transmitted by the tissue, and to transmit the filtered light.

Importantly, the filtered light is differently polarized compared to the emitted light (e.g., perpendicularly polarized). In other words, the first polarization filter 120 and second polarization filter 130 polarize/filter light differently to each other.

The first and second polarization filter 130 may be separate components. Alternatively, the first and second polarization filters 120/130 may be different parts of the same component. For example, the first and second polarization filters 120/130 may be a circular polarizer composed by two, independently polarizable, annular concentrical parts forming the entire circle. The external filter may overlap with the light source 110, while an internal filter overlaps with the light sensor 140. This may enable a more compact system.

Moreover, the first (and second) polarization filter may be adapted to be operable in one or more (a plurality) of different polarization modes. In each polarization mode, the polarization filters may have different modes of polarization (i.e., they filter the light differently). This enables multi-polarization imaging.

In other words, the first polarization filter 120 is adapted to be operable in a plurality of first polarization modes, wherein a polarization characteristic of the transmitted polarized light is different in each first polarization mode. Further, the second polarization filter 130 is adapted to be operable in a plurality of second polarization modes, wherein a polarization characteristic of the filtered light is different in each second polarization mode.

The system 100 may optionally comprise a first lens element 122 and/or a second lens element 132 for appropriately directing light illuminating the tissue and backscattered and/or transmitted by the tissue, respectively. The lens elements may be used simply to appropriately focus the light to the appropriate component/tissue, or may be used to selectively direct the light to/from the tissue.

While depicted as separate components in Fig. 1, the first lens element 122 and second lens element 132 may be fulfilled by a single component/device. Furthermore, the first lens element 122 and the second element may be provided in the light path before or after the polarization filters.

Accordingly, the controller 150 is configured for controlling the operation of the above-mentioned components. Specifically, at its simplest, the controller 150 is configured to control the light source 110 to emit first light having a first wavelength and control the light sensor 140 to detect first filtered light corresponding to the first light. That is, the controller 150 prompts the light source 110 to emit light toward the tissue (via the first polarization filter 120) and prompts the light sensor 140 to detect the emitted light (after being transmitted through the first polarization filter 120, backscattered and/or transmitted by the tissue, and transmitted through the second polarization filter 130.

Subsequently, the processor 160 is configured to process the first filtered light to generate an image of the tissue of the subject. In other words, the processor 160 processes/analyses a signal describing the light detected by the light sensor 140 in order to generate an image of (at least part) of the tissue of the subject.

Of course, the controller 150 may prompt the light source 110 and light sensor 140 to perform the above many times. This may be performed for various parts of the tissue of the subject, and the resultant light signal used to produce a complete (or more complete) image of the tissue of the subject.

More specifically, the controller 150 may be further configured to control the light source 110 to emit second light having a second wavelength different from the first wavelength, control the light sensor 140 to detect second filtered light corresponding to the second light. That is, the tissue is illuminated with polarized light of two different wavelengths (or in two narrow wavelength bands), and the light sensor 140 detects the two different wavelengths of light.

The processor 160 is further configured to process the first filtered light and the second filtered light (or signals describing the first filtered light and the second filtered light) to generate the image of the tissue of the subject.

In order to implement SDPI, the processor 160 may be configured to process the first filtered light to generate a first intermediate image of the tissue of the subject, process the second filtered light to generate a second intermediate image of the tissue of the subject, and generate the image of the tissue of the subject based on a difference between the first intermediate image and the second intermediate image. Accordingly, a signal-to-noise ratio of the image of the tissue may be improved.

The selected wavelengths may be dependent on a predetermined sequence of wavelengths. That is, there may be certain wavelengths that may be used to illuminate the tissue. This may be dependent on the tissue/anatomy/structure of interest, including the depth and spatial resolution required. The controller 150 is configured to wavelengths based on the predetermined sequence (i.e., the controller 150 may consult memory to determine the wavelengths).

In addition, or alternatively, the processor 160 may be configured to process the first filtered light to generate Stokes vectors representing the polarization of the first filtered light. The Stokes vectors may then be analyse/processed using Mueller matrix decomposition to determine a characteristic value describing a property of the tissue of the subject. Said characteristic value may describe structures of the tissue and may be used for future selection of wavelengths and/or polarizations of lights used for imaging.

Furthermore, the processor 160 may be configured to process the first filtered light (i.e., a signal describing the first filtered light) to determine an object detection value indicative of the presence of an object within the tissue of the subject. This may be performed by an image processing algorithm configured to determine (irregular) objects in an image corresponding to the first filtered light.

Subsequently, and responsive to the object detection value determined by the processor 160 meeting a predetermined requirement, the controller 150 may control the light source 110 to emit a plurality of lights, each having a different wavelength, and control the light sensor 140 to detect a plurality of filtered lights corresponding to the plurality of lights with different wavelengths. That is, the controller 150 may control the light source 110 and light sensor 140 to perform an extensive scan of the tissue corresponding to the object.

The processor 160 may then process the plurality of filtered lights to generate a detailed image of the tissue of the subject, the detailed image having a greater spatial resolution than the image of the tissue of the subject. That is, by imaging the tissue with a plurality of different wavelengths, an overall image having improved spatial resolution than an image based on light of one wavelength, may be obtained.

Alternatively, or in addition to the above, responsive to the object detection value determined by the processor 160 meeting a predetermined requirement the control unit may be configured to control the light source 110 to emit a plurality of lights, each having a different polarization characteristic. Of course, the control unit may also control the light sensor 140 to detect a plurality of filtered lights corresponding to the plurality of lights with different polarization characteristics. This may require either multiple polarizers, or a capability to manipulate the polarizers (using known methods like rotation, or by manipulation of liquid crystals).

Similarly to the above, the processor 160 may be further configured to process the plurality of filtered lights to generate a detailed image of the tissue of the subject, the detailed image having a greater spatial resolution than the image of the tissue of the subject.

It should be appreciated that a combination of the two above methods (of multiple different wavelengths and multiple different polarizations of light used to image the tissue) may be employed in order to generate an improved image also.

Finally, the system may (optionally) also comprise an analysis unit. The analysis unit is configured to identify features (e.g., structures, anatomies, calcifications and tumors) present in the image of the tissue of the subject. The analysis unit then maps the identified features to a model representing an anatomy of the subject including the tissue to generate a subject-specific feature map. The model may be obtained from memory or may be generated by the analysis unit.

In a complex example, the analysis unit may be further configured to compare the subject-specific feature map to a previous subject-specific feature map representing a mapping of previously identified features of the tissue of the subject. Based on the result of the comparison, a change in the features of the subject may be identified. For example, a rate of growth of a tumor may be identified, or the releasing of blockages of a duct noted.

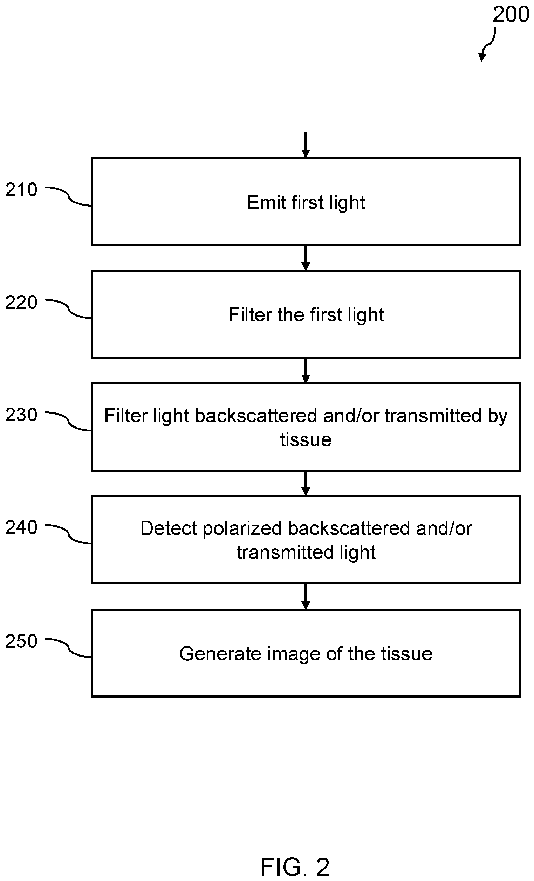

Fig. 2 presents a method for imaging tissue of a subject according to an embodiment of the invention.

In step 210, first light having a first wavelength is emitted toward the tissue of the subject. The first light is monochromatic, in that it either has one wavelength or a thin/narrow band of wavelengths. That is, first light is used to illuminate the tissue of the subject.

In step 220, the first light is filtered, such that the first light is polarized. This is performed prior to the first light being incident on the tissue of the subject. As a result, monochromatic, polarized light illuminates (and penetrates) the tissue of the subject.

In step 230, light that is backscattered and/or transmitted by the tissue is filtered, such that the backscattered and/or transmitted light is polarized. The backscattered and/or transmitted light is differently polarized compared to the first light.

To paraphrase, light is emitted, passed through a filter so as to have a first polarization, is reflected/backscattered and/or transmitted by the tissue, and passed through another filter so as to have a second polarization (different to the first).

In step 240, the polarized backscattered and/or transmitted light is detected. This may comprise measuring said light and converting the light into a signal describing the polarized backscattered and/or transmitted light.

Finally, in step 250, the detected light is processed/analysed to generate an image of the tissue of the subject. Of course, some of the steps above may be repeated with different wavelengths and/or polarizations of light, and at different parts of the tissue.

A detailed explanation of an implementation of control of the invention will now be provided.

Specifically, a light sensor (e.g., a camera) is provided with appropriate optics (such as a CMOS sensor) to image the subject's tissue under both visible, IR and/or NIR lighting. The light sensor may be any sensor currently used to detect light in such wavelength bands.

Images may be captured by the light sensor when the system is handled (i.e., when the system is integrated in the personal care device, the images may be captured responsive to the device being used/handled). It may be determined whether the tissue/anatomy of interest is in the field of view of the image sensor through an image processing or AI module (e.g., nipple recognition, breast recognition etc. by a deep learning algorithm). Of course, while breast and nipple tissue are of particular interest, it should be understood the proposed sub-surface imaging technique allows for imaging of any type of tissue in any anatomical part.

Once the anatomy has been detected in the field of view of the light sensor, a progressive scanning protocol may be activated. The progressive scanning protocol may sequentially increase the depth of imaging (D) using the information from the (relatively quick) SPDI to improve a Mueller decomposition. Also, protocol may use the detailed optical properties obtained from the Mueller decomposition to improve the quality of the SPDI image. The nature of the improvement for the Mueller matrix decomposition is due to taking into account the optical properties of the tissue under examination; this knowledge improves substantially the Mueller matrix decomposition possibility and returns a much better signal to noise ratio profile for the image. For the SPDI the detailed tissue information gained from the results of the Mueller matrix decomposition imaging for the previous tissue layer, at depth D, empowers the imaging method to increase its signal to noise ratio, at depth D+1, because the first frequencies' polarimetric image details, of the two, required by the SPDI imaging are known and much better detailed than it would be by the simpler imaging used in the SPDI method alone.

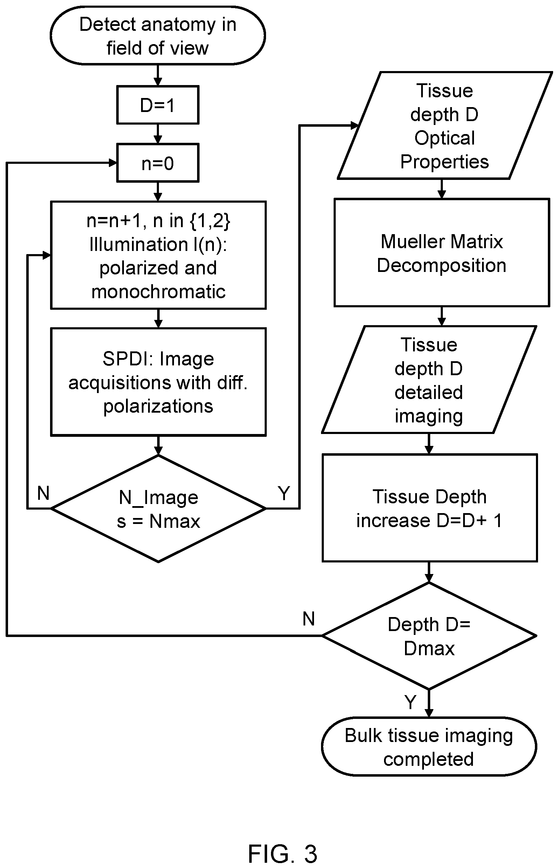

This process is summarised in Fig. 3, which depicts the main phases of an imaging procedure for increasing depths.

For the illustrated SPDI image acquisition part, first the VISNIR illumination component (i.e., the light source) is activated. This component, under the control of a general progressive multi-polarization and multi-frequency imaging sequence algorithm, starts illuminating the subject's tissue with a series of frequencies (depending on the tissue to be imaged). In the case of the breast, this sequence algorithm may begin in the higher part of the NIR spectrum, to gain deeper tissue penetration (e.g., 800nm and 1000nm are used in sequence to illuminate the subject). Of course, this light is also polarized, with a filter after the frequency selection filter/monochromator, in a given direction (e.g., horizontally, vertically).

In simple embodiments, the invention uses only the reflected/backscattered light, and consequently the reflected light is collected, in order, by a polarization filter that is perpendicularly polarized to the one used to illuminate the subject; the collected light is then fed into the camera's optics and an image is acquired. Alternatively, light that has been transmitted may also be collected, but this may require additional components and/or more complex optics.

The same procedure may be followed to acquire a second image at the second nearby frequency (e.g., 1000nm if the previous image was acquired illuminating with a frequency of 800nm). The two, initial, acquired images are then subtracted to give as a result the SPDI image. In this image the presence of semi-solid (e.g., calcifications, blocked ducts etc.) structures are enhanced and made more visible by the selection of the photons bringing the most information while eliminating the majority of the back-scattered ones that underwent heavy scattering inside the tissue or have been just back-scattered from the tissue surface (i.e., skin).

In some cases, feedback from the reflected/backscattered light may be automatically leveraged to compensate for modification in the light signal delivery to the tissue (for example, the reflected light may indicate environmental changes, such as inclination etc.). The process is repeated for several different polarizations to also increase the horizontal resolution of the SPDI images. It should be noted that the opportunity to increase such resolution depends on the imaged tissue. For example, this may be particularly useful for imaging internal smaller ducts of nipples.

Once the first SPDI imaging phase has been completed, the system switches to Mueller decomposition mode. The main change in this mode is in the polarization filters, which allow for a more complex series of polarizations and frequencies to be used both to illuminate the tissue, and to read the backscattered/reflected light filtering many polarization directions.

However, the optical characteristics deduced during the SPDI phase are used in the Mueller analysis to speed up and ensure robust decomposition (because the knowledge of the tissue characteristics can be embedded in the decomposition algorithm). For example, for imaging nipple ducts, the decomposition focuses mostly on the retardance component of the matrix since it is demonstrated to be much more suitable for imaging based on the spatial orientation of the tissue structures. Indeed, the ducts form a structure akin to a bundle of fibres that show a clear spatial orientation, making this particular decomposition well suited for this imaging task.

The polarization changes required by this imaging phase can be obtained by polarization filters including technologies such as liquid crystals (LCs) or photo elastic modulators (PEMs), by mechanically by rotating linear polarizers, wire grid polarizers and quarter wave-plates, or by diffraction using polarizing prisms, gratings, or graded index lenses.

The lateral resolution (Full Width at Half Maximum - FWHM of point spread function of the polarization preserving photons in tissue for an incident beam) is ~200 µm in the visible wavelength range and for typical tissue properties. This FWHM is very well suited for the imaging of the nipples' ducts to a depth greater than 2mm (which they commonly meet or exceed).

The light source (i.e., broadband LED) used for some embodiments of the invention may output light in a spectrum approximately from 450nm to 1200nm. Therefore, the range of subsequent, close frequencies usable by the invention to illuminate the subject are included in this range. Initially the invention, may begin the scanning with a difference between the two frequencies of about 200nm. In this case of breast tissue, this enables an approximate resolving power of about 5mm (from approx. tissue depth of 1,5 to 1cm), which is the depth and size of the calcifications most commonly found in the breasts. The ideal frequencies involved in the initial scan, and their separation, change for imaging different types of tissue given the size of the object that may be detected, or the depth of interest.

It remains to be said that the sampling of the images of the tissue may be guided by an anisotropic spatial sampling dictated by the spatial distribution of structures of interest. For example, tumors in breast tissue are known to be more likely to develop in certain areas of the breast than others. In practice, this means that the field of view of the camera is sampled according to known statistics.

The data so acquired (SPDI and Mueller decomposition images, and corresponding frequencies) may be saved and connected to a 3D model of the tissue of the subject that has been imaged. This enables the analysis of the temporal evolution of imaged structures and the, eventual, enrichment obtained using frequencies with lower spacing to gain better depth resolution. An AI module may be tasked with the analysis, both on-line and off-line, of such acquired data to detect possible abnormalities.

In a specific example, the 3D model of the anatomy may be obtained for the breasts by an AI module applied to the image captured when the breast is detected in the field of view of the camera. The 3D model may also be acquired for the nipples by the same AI module as in the breasts since an image of the global anatomy is acquired also in this case to trigger the polarimetric analysis. In this case, though, the model is further enriched by the use of the much more detailed images acquired since the limited size of the average nipples (0.6 cm height by 1.2cm diameter) allows for a complete scanning of the whole volume of the anatomy from side to side in subsequent acquisition sessions.

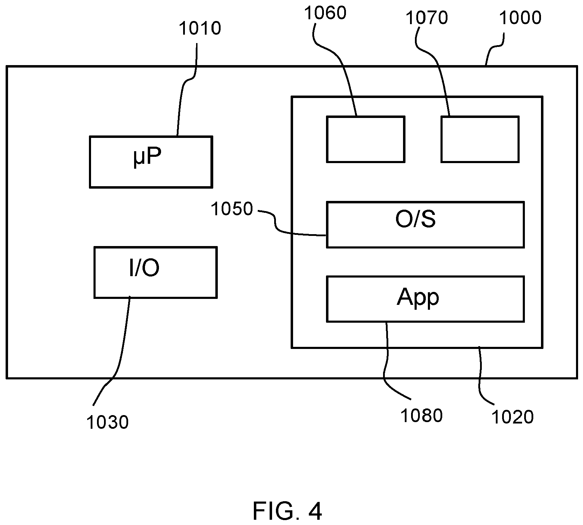

Fig. 4 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g., connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, functional programming and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs. 2 and 3, and the system described in relation to Fig. 1, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out a method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram Fig. 1 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A system (100) for imaging tissue of a subject, comprising:
a light source (110) configured to emit monochromatic light toward the tissue of the subject;
a first polarization filter (120) configured to polarize light emitted by the light source, and to transmit polarized light toward the tissue of the subject;
a second polarization filter (130) configured to filter light backscattered and/or transmitted by the tissue, and to transmit the filtered light, wherein the filtered light is differently polarized compared to the emitted light;
a light sensor (140) configured to detect the filtered light;
a controller (150) configured to:
control the light source to emit first light having a first wavelength; and
control the light sensor to detect first filtered light corresponding to the first light; and
a processor (160) configured to process the first filtered light to generate an image of the tissue of the subject.

2. The system of claim 1, wherein the controller (150) is further configured to:
control the light source (110) to emit second light having a second wavelength different from the first wavelength; and
control the light sensor (140) to detect second filtered light corresponding to the second light, and
the processor (160) is further configured to process the first filtered light and the second filtered light to generate the image of the tissue of the subject.

3. The system of claim 2, wherein the controller (150) is further configured to determine the second wavelength different from the first wavelength based on a predetermined sequence of wavelengths.

4. The system of claim 2 or 3, wherein the processor (160) is configured to:
process the first filtered light to generate a first intermediate image of the tissue of the subject;
process the second filtered light to generate a second intermediate image of the tissue of the subject; and
generate the image of the tissue of the subject based on a difference between the first intermediate image and the second intermediate image.

5. The system of any of claims 1 to 4, wherein the processor (160) is further configured to:
process the first filtered light to generate Stokes vectors representing the polarization of the first filtered light; and
analyse the Stokes vectors using Mueller matrix decomposition to determine a characteristic value describing a property of the tissue of the subject.

6. The system of any of claims 1 to 5, wherein the first polarization filter (120) is adapted to be operable in a plurality of first polarization modes, wherein a polarization characteristic of the transmitted polarized light is different in each first polarization mode, and
wherein the second polarization filter (130) is adapted to be operable in a plurality of second polarization modes, wherein a polarization characteristic of the filtered light is different in each second polarization mode.

7. The system of any of claims 1 to 6, wherein the light source (110) comprises:
a light emitter (112) configured to emit light in the infrared, near-infrared, and/or visible light spectrums; and
a variable light frequency filter (114) configured to receive the emitted light, and output monochromatic light toward the tissue of the subject, and
wherein the controller (150) is configured to control the variable light frequency filter to output monochromatic light having the first wavelength as the first light.

8. The system of any of claims 1 to 7, wherein the processor (160) is further configured to process the first filtered light to determine an object detection value indicative of the presence of an object within the tissue of the subject.

9. The system of claim 8, wherein the controller (150) is configured to, responsive to the object detection value determined by the processor meeting a predetermined requirement:
control the light source (110) to emit a plurality of lights, each having a different wavelength; and
control the light sensor (140) to detect a plurality of filtered lights corresponding to the plurality of lights with different wavelengths, and
wherein the processor (160) is further configured to process the plurality of filtered lights to generate a detailed image of the tissue of the subject, the detailed image having a greater spatial resolution than the image of the tissue of the subject.

10. The system of claim 8 or 9, wherein the controller (150) is configured to, responsive to the object detection value determined by the processor meeting a predetermined requirement:
control the light source (110) to emit a plurality of lights, each having a different polarization characteristic; and
control the light sensor (140) to detect a plurality of filtered lights corresponding to the plurality of lights with different polarization characteristics, and
wherein the processor (160) is further configured to process the plurality of filtered lights to generate a detailed image of the tissue of the subject, the detailed image having a greater spatial resolution than the image of the tissue of the subject.

11. The system of any of claims 1 to 10, further comprising an analysis unit (170) configured to:
identify features present in the image of the tissue of the subject; and
map the identified features to a model representing an anatomy of the subject including the tissue to generate a subject-specific feature map.

12. The system of claim 11, wherein the analysis unit (170) is further configured to:
compare the subject-specific feature map to a previous subject-specific feature map representing a mapping of previously identified features of the tissue of the subject; and
identify a change in features of the subject based on a result of the comparison.

13. The system of any of claims 1-12, wherein the tissue is breast tissue of the subject, and wherein the system (100) is mounted on a breast pump device.

14. A method (200) for imaging tissue of a subject, comprising:
emitting (210) a first light having a first wavelength toward the tissue of the subject,
wherein the first light is monochromatic;
filtering (220) the first light such that the first light is polarized;
filtering (230) light that is backscattered and/or transmitted by the tissue, such that the backscattered and/or transmitted light is polarized, wherein the backscattered and/or transmitted light is differently polarized compared to the first light;
detecting (240) the polarized backscattered and/or transmitted light; and
processing the detected light to generate (250) an image of the tissue of the subject.

15. A computer program product comprising computer program code means which, when executed on a computing device having a processing system, cause the processing system to perform all of the steps of the method according claim 14.
